# EUROPÄISCHE PATENTSCHRIFT

(11) **EP 2 792 301 B1**
(45) Veröffentlichungstag und Bekanntmachung des Hinweises auf die Patenterteilung: **15.08.2018**
(21) Anmeldenummer: 14163825.4
(22) Anmeldetag: 08.04.2014
(51) Int. Cl.: A61B 5/107

(54) **Vorrichtung und Verfahren zur Bestimmung des Umfangs eines Körperteils**
Device and method for determining the circumference of a body part
Dispositif et méthode de détermination de la dimension d'une partie du corps

(30) Priorität: 17.04.2013 DE 102013206965
(43) Veröffentlichungstag der Anmeldung: 22.10.2014
(73) Patentinhaber: Pohlig GmbH, 83278 Traunstein (DE)
(72) Erfinder: Pohlig, Kurt, 83278 Traunstein (DE); Stahl, Josef, 5091 Unken (AT)
(74) Vertreter: Pfenning, Meinig & Partner mbB

(56) Entgegenhaltungen:
- WO-A1-02/053032
- WO-A2-2007/095906
- WO-A2-2007/112527
- DE-C1- 3 833 669

## Beschreibung

Die vorliegende Erfindung betrifft eine Vorrichtung zur Bestimmung des Umfangs eines Körperteils.

Im Bereich der Orthopädietechnik bei der Herstellung von individuellen Orthesen oder Prothesen wird oft der Umfang des von der Orthese oder der Prothese aufzunehmenden Körperteils an verschiedenen Stellen des Körperteils bestimmt. Die Umfangswerte fließen dabei in die Ausbildung der individuellen Form des Orthesen- oder Prothesenschafts ein. Eine genaue Bestimmung der Umfangswerte ist dabei notwendig um später einen optimalen Sitz der Orthese oder der Prothese an dem entsprechenden Körperteil zu gewährleisten und somit optimale Therapieergebnisse bzw. einen optimalen Tragekomfort zu erhalten.

Bei der Umfangsbestimmung wird das Körperteil in einem bestimmten Maße an der Messstelle zusammengedrückt um den Druck bei angelegter Orthese oder Prothese zu simulieren. Bei der Umfangsbestimmung mittels eines Maßbands legt ein Orthopädietechniker daher das Maßband unter Ausübung einer selbst gewählten Zugkraft um das Körperteil und liest dann den gemessenen Wert ab.

Problematisch hierbei ist jedoch, dass die Ausübung der Zugkraft nicht mit einem vordefinierten Wert erfolgt, sondern von Messung zu Messung und vom messenden Orthopädietechniker abhängig variiert. So wird beispielsweise der Umfang in verschiedenen Bereichen des Körperteils und von verschiedenen Orthopädietechnikern mit unterschiedlichen Zugkräften gemessen. Dies hat zur Folge, dass ein mit auf diese Weise gemessenen Umfangswerten angepasster Prothesen- oder Orthesenschaft nicht gleichmäßig am Körperteil sitzt und eine optimale Funktion der Prothese oder der Orthese nicht erreicht wird.

Des Weiteren lassen sich die auf diese Weise ermittelten Umfangswerte auch nicht ohne Weiteres reproduzieren.

Die DE 38 33 669 C1 offenbart eine Meßeinrichtung zum Messen des Umfanges eines elastischen Körpers. Ein flexibles, undehnbares Meßband trägt eine Meßskala. Ein erstes freies Ende des Meßbandes ist an einem ersten Führungsteil derart längsverschieblich geführt, daß die Meßskala an einer ersten Marke des ersten Führungsteiles vorbeiläuft. Eine Feder zum Erzeugen einer vorbestimmten Zugspannung des um den Körper gelegten Meßbandes greift an dem ersten Führungsteil an. Um die vorbestimmte Zugspannung über die Länge des Meßbandes konstant zu halten, ist das erste Führungsteil am zweiten freien Ende des Meßbandes fest angeschlagen. Das Meßband ist vom zweiten freien Ende fort zunächst an einem zweiten Führungsteil und dann am ersten Führungsteil geführt. Die Feder ist zwischen den Führungsteilen angeordnet. Einstellmittel sind vorgesehen, um die Führungsteile in einem Abstand voneinander anzuordnen, der größer als deren Abstand im ungespannten Zustand der Feder ist. Das Meßband ist an den Führungsteilen fixierbar.

Die WO 02/053032 offenbart ein Meßgerät zur genauen Erfassung von Umfangsmaßen, insbesondere von Körperteilen bzw. -bereichen, wobei in einem Gehäuse ein flexibles Maßband vorgesehen ist, das mittels einer Feder in das Gehäuse rückholbar ist, wobei das freie Ende des flexiblen Maßbandes mit einer Einklinkvorrichtung versehen ist, welche an einem am Gehäuse vorgesehenen Gegenstück bzw. an dem aus dem Gehäuse herauskommenden Bereich des Maßbandes lösbar einklinkbar ist, und wobei eine elektronische Maßabnahmeeinrichtung vorgesehen ist, die den gemessenen Wert über ein Display oder dergleichen anzeigt.

Die WO 2007/112527 A2 offenbart eine Vorrichtung und ein Verfahren zur Messung von Expansion und Kontraktion im Allgemeinen und insbesondere zur Überwachung der Atmung von Patienten durch Messung des Torso-Perimeters (Thorax-Cirtometrie) in Echtzeit, insbesondere zur Messung der Thorax-Expansibilität bei Patienten unter Physiotherapie oder klinischen Behandlung oder in jedem Fall, die die Bewertung der Beweglichkeit des Thorax erfordert. Die Messung erfolgt über einen Widerstandssensor, der ein Spannungssignal erzeugt, das proportional zur Position eines Gleitcursors ist. Andere Ausführungsformen können auch einen kapazitiven Sensor und einen linearen Codierer verwenden.

Die Aufgabe der vorliegenden Erfindung ist es daher, eine möglichst einfache Vorrichtung bereitzustellen, die es ermöglicht vergleichbare und reproduzierbare Werte für den Umfang eines Körperteils zu bestimmen. Eine weitere Aufgabe der Erfindung ist es, ein einfaches und schnelles Verfahren zur Bestimmung von vergleichbaren und reproduzierbaren Werten für den Umfang eines Körperteils anzugeben.

Die Aufgabe wird durch eine Vorrichtung nach Anspruch 1 sowie ein Verfahren nach Anspruch 11 gelöst. Vorteilhafte Weiterbildungen der erfindungsgemäßen Vorrichtung sowie des erfindungsgemäßen Verfahrens werden in den jeweiligen abhängigen Ansprüchen angegeben.

Eine erfindungsgemäße Vorrichtung zur Bestimmung des Umfangs eines Körperteils weist einen Grundkörper und ein gegenüber dem Grundkörper in einer ersten Richtung verschiebbar, vorteilhafterweise am oder im Grundkörper, gelagertes Zugelement auf. Die Vorrichtung weist ferner ein Band auf, dessen erstes Ende an einem Referenzpunkt des Grundkörpers fest mit dem Grundkörper verbunden ist und dessen zweites Ende an einer Arretierungsstelle des Zugelements an dem Zugelement arretierbar ist.

Unter einem Band sind hier sowohl Bänder mit einem im Wesentlichen rechteckigen oder ovalen Querschnitt als auch Schnüre mit einem im Wesentlichen kreisförmigen Querschnitt zu verstehen.

Ein weiterer Bestandteil der erfindungsgemäßen Vorrichtung ist eine Messvorrichtung, mit der die Länge des Bandes zwischen dem Referenzpunkt und der Messstelle direkt oder indirekt bestimmbar ist. Aus der Länge des Bandes ist dann der Umfang eines von dem Band umfassten Körperteils bestimmbar.

Die erfindungsgemäße Vorrichtung weist weiterhin eine Zugerzeugungsvorrichtung auf, die zur Ausübung einer von der Verschiebung des Zugelements im Wesentlichen unabhängigen einheitlichen Zugkraft parallel zu der ersten Richtung über das Zugelement auf das Band eingerichtet ist. Die erfindungsgemäße Vorrichtung zeichnet sich dadurch aus, dass die Zugerzeugungsvorrichtung eine Gasdruck- oder eine Öldruckfeder aufweist.

Mit der erfindungsgemäßen Vorrichtung ist es möglich den Umfang eines Körperteils auf einfache und schnelle Art und Weise zu bestimmen. Eine Gasdruckfeder ermöglicht eine konstante Zugkraft entlang des gesamten Federwegs. Die mit der erfindungsgemäßen Vorrichtung erhaltenen Werte für den Umfang sind weder von der Messung noch vom messenden Orthopädietechniker abhängig und somit reproduzierbar und vergleichbar.

In einer weiteren Ausgestaltung, die jedoch nicht Teil der Erfindung ist, kann die Zugerzeugungsvorrichtung eine Feder aufweisen.

Es ist auch möglich eine Schrauben- oder eine Elastomerfeder als Feder in der Zugerzeugungsvorrichtung zu verwenden.

Die Zugerzeugungsvorrichtung kann derart ausgebildet sein, dass sie eine Kraft zwischen 5 N und 80 N, vorteilhafterweise zwischen 5 N und 20 N, vorteilhafterweise von 10 N auf die Zugvorrichtung ausüben kann. In einer besonders bevorzugten Ausgestaltung der Erfindung ist die von der Zugerzeugungsvorrichtung ausgeübte Kraft einstellbar und zwischen verschiedenen

Werten variierbar. Hierdurch ist es möglich den Umfang eines Körperteils unter Ausübung verschiedener Zugkräfte auf das Band zu messen. Insbesondere ist es dadurch möglich die auf das Band ausgeübte Zugkraft abhängig von der zu messenden Körperregion einzustellen.

In einer weiteren vorteilhaften Ausgestaltung kann der Grundkörper als länglicher Hohlkörper, insbesondere als Hohlzylinder, mit einem ersten und einem dem ersten Ende gegenüberliegenden zweiten Ende ausgebildet sein. An dem ersten Ende des als länglicher Hohlkörper ausgebildeten Grundkörpers kann das erste Ende des Bands befestigt sein. Das zweite Ende des als länglicher Hohlkörper ausgebildeten Grundkörpers kann eine Öffnung aufweisen, deren Querschnittsfläche senkrecht zur Längsachse des Grundkörpers orientiert sein kann. Die Zugerzeugungsvorrichtung kann dann derart in dem Grundkörper gelagert sein, dass die Richtung der Zugkraft und die Längsachse des als länglicher Hohlkörper ausgebildeten Grundkörpers parallel zueinander liegen.

Vorteilhafterweise kann auch das Zugelement länglich ausgebildet sein und zumindest teilweise innerhalb des Grundkörpers in Richtung von dessen Längsachse durch die Öffnung am zweiten Ende des Grundkörpers hindurch verschiebbar gelagert sein. Der Grundkörper und das Zugelement bilden so zwei gegeneinander verschiebbare Teile. Insbesondere können der Grundkörper und das Zugelement auch als Linearführungen beispielsweise in Form von U-förmigen Hohlprofilen ausgebildet sein. Das längliche Zugelement kann dann durch die Zugerzeugungsvorrichtung mit einer Druckkraft relativ zum Grundkörper beaufschlagbar sein.

Die Vorrichtung kann weiter dahingehend ausgebildet sein, dass sie eine Verriegelungseinrichtung zur Arretierung des Zugelements in einem vorgespannten Zustand aufweist, in dem das Zugelement keine Zugkraft auf das Band ausübt. Die Verriegelungseinrichtung kann ein Entriegelungselement aufweisen, durch welches das Zugelement von dem vorgespannten Zustand in einen Zustand bringbar ist, in dem das Zugelement eine Zugkraft auf das Band ausübt.

Als Verriegelungseinrichtung kann in einer Öffnung der Wand des Grundkörpers ein senkrecht zur Längsausdehnung des Grundkörpers federnd gelagerter Stift angeordnet sein, der senkrecht gegen das Zugelement oder in eine Einkerbung des Zugelements drückt. Als Entriegelungselement dient der Stift. Durch einen von dem Grundkörper weggerichteten Zug an dem Stift kann dann die Verriegelungseinrichtung entriegelt werden.

Alternativ können als Verriegelungseinrichtung sowohl an dem Grundkörper als auch an dem Zugelement zwei flächige, längliche Bügel fest angeordnet sein. Diese Bügel sind vorzugsweise parallel zueinander und überlappen an ihren einander zugewandten Enden, wenn das Zugelement in den Grundkörper hineingedrückt wird. Die einander zugewandten Enden sind ineinander verrastbar, so dass die Vorspannung des Zugelements erhalten bleibt. Als Entriegelungselement dient hierbei ein Hebel, der in Richtung senkrecht zur Längsachse des Grundkörpers an einem der Bügel zur Lösung der Verrastung angeordnet ist.

In einer bevorzugten Ausgestaltung der Erfindung kann das Band austauschbar an der Vorrichtung befestigt sein.

An dem Zugelement kann außerdem eine Klemme zum Arretieren des zweiten Endes des Bands fest angeordnet, angeschraubt und/oder angenietet sein. Die Klemme kann ferner in Ihrer Breite variierbar ausgestaltet sein, sodass Bänder verschiedener Breiten in der Vorrichtung verwendbar sind.

Zur Führung des Bandes kann der Grundkörper Führungsrollen oder Gleitflächen aufweisen. Die Führungsrollen oder Gleitflächen können ebenfalls in Ihrer Breite variierbar ausgestaltet sein, um Bänder verschiedener Breiten führen zu können. Alternative kann eine Führungsrolle in ihrem mittleren Bereich bezüglich der Drehachse eingeschnitten sein, um zwei verschiedene Bänder verschiedener Breite optimal führen zu können.

Bei dem verwendeten Band kann es sich insbesondere um ein Maßband handeln. Anstelle eines Maßbandes kann jedoch auch eine Schnur oder eine Schnur mit einer Messskala eingesetzt werden. Wird ein Band oder ein Maßband verwendet, so kann dessen Breite zwischen 15 mm und 20 mm, vorteilhafterweise 15 mm, vorteilhafterweise 20 mm betragen. Die Breite des Bandes wird dabei jeweils abhängig von der zu messenden Körperregion gewählt.

In einer weiteren Ausführungsform kann die Messvorrichtung eine digitale Messeinheit zur Bestimmung der Länge des Bandes aufweisen. Ebenso denkbar ist es, dass die Messvorrichtung eine markierten Messstelle aufweist. Bei Verwendung eines Maßbandes oder einer Schnur mit Messskala als Band kann dann die Länge des Bandes an der Messstelle abgelesen werden.

Erfindungsgemäß ist auch ein Verfahren zur Bestimmung eines Umfangs eines Körperteils mit einer erfindungsgemäßen Vorrichtung vorgesehen. Bei diesem Verfahren wird zunächst das Band um ein Körperteil gelegt. Danach wird das zweite Ende des Bands an dem Zugelement derart arretiert, dass das Band an dem Körperteil anliegt. Anschließend wird mittels der Zugerzeugungsvorrichtung über das Zugelement eine Zugkraft auf das Band ausgeübt und mittels der Messvorrichtung die Länge des Bands zwischen dem Referenzpunkt und der Messstelle und daraus der Umfang des Körperteils bestimmt.

Das Verfahren kann weiter dahingehend ausgestaltet sein, dass die Zugerzeugungsvorrichtung vor dem Anlegen des Bandes an das Körperteil vorgespannt und mittels der Verriegelungseinrichtung arretiert wird. Zur Aufbringung der Zugkraft auf das Band wird dann das Entriegelungselement betätigt.

Im Folgenden wird ein konkretes Beispiel einer erfindungsgemäßen Vorrichtung anhand von Figuren beschrieben. Die in dem konkreten Beispiel beschriebenen Merkmale sind dabei nicht nur auf das Beispiel beschränkt, sondern können auch unabhängig von diesem realisiert sein.

Es zeigen
- Figur 1: eine erfindungsgemäße Vorrichtung in einer Perspektivansicht,
- Figur 2: eine erfindungsgemäße Vorrichtung in einer Seitenansicht.

In der nachfolgenden Beschreibung der Figuren bezeichnen gleiche oder ähnliche Bezugszeichen gleiche oder ähnliche Elemente. Folgende Bezugszeichen wurden verwendet:

| | |
|---|---|
| Grundkörper | 1 |
| Zugelement | 2 |
| Band | 3 |
| erstes Ende des Bandes | 3a |
| zweites Ende des Bandes | 3b |
| Referenzpunkt | 4 |
| Arretierungsstelle | 5 |
| Messkante | 6 |
| Öffnung | 8 |
| Rollenkasten | 9 |
| Führungsrolle | 10 |
| Bandlaufrolle | 11 |
| Verriegelungseinrichtung | 12 |
| Klemme | 13 |
| Griffbolzen | 14, 15 |
| Bügel | 16 |

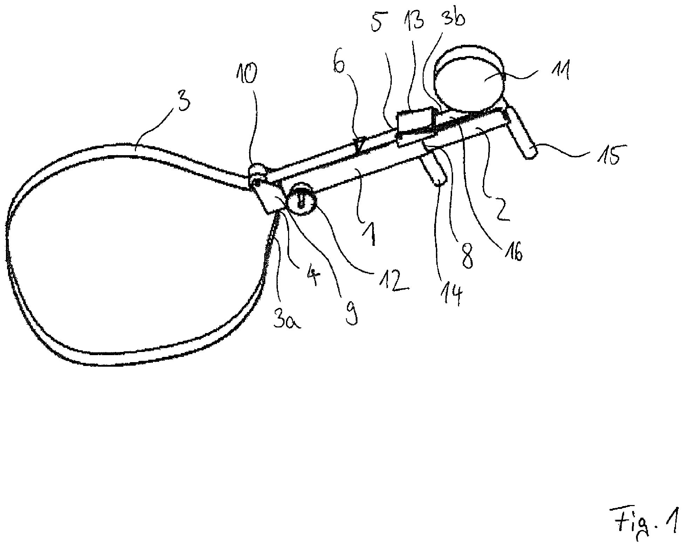

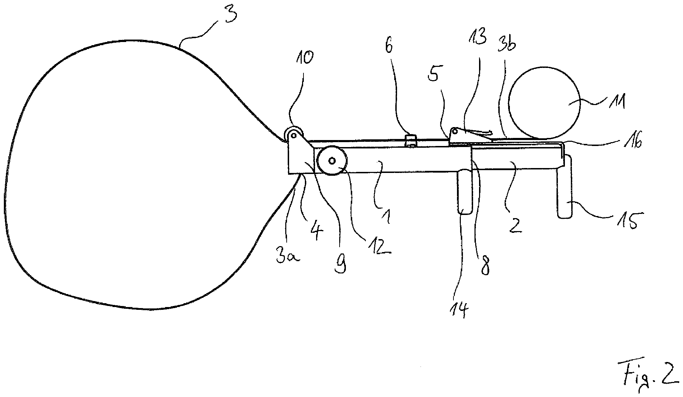

Figur 1 und 2 zeigen eine Vorrichtung gemäß einer konkreten Ausführungsform der vorliegenden Erfindung in einer Perspektivansicht (Figur 1) und einer Seitenansicht (Figur 2).

Die Vorrichtung weist einen Grundkörper 1 auf, der im Wesentlichen als Hohlzylinder ausgebildet ist. Der hohlzylinderförmige Grundkörper 1 weist an einem ersten Ende einen Rollenkasten 9 und an einem dem ersten Ende gegenüberliegenden zweiten Ende eine Öffnung 8 von der Größe des Umfangs des hohlzylinderförmigen Grundkörpers 1 auf. In dem Grundkörper 1 ist ein ebenfalls zylinderförmiges Zugelement 2 entlang der Längsachse des Grundkörpers 1 relativ zum Grundkörper 1 durch die Öffnung 4 des Grundkörpers 1 hindurch verschiebbar gelagert. In dem Zugelement 2 ist eine Gasdruckfeder (hier nicht gezeigt) gelagert, die im Inneren des Grundkörpers 1 gegen den Rollenkasten 9 und das Zugelement 2 drückt. Benachbart zum Rollenkasten 4 ist eine Verriegelungseinrichtung 12 seitlich am Grundkörper 1 zur Arretierung der Vorspannung der Gasdruckfeder bzw. des Zugelements 2 angeordnet. Im Bereich der Öffnung 4 des Grundkörpers 1 sowie an der der Öffnung 4 abgewandten Seite des Zugelements 2 sind Griffbolzen 14 und 15 senkrecht zur Längsachse des Grundkörpers 1 und parallel zueinander am Grundkörper 1 bzw. am Zugelement 2 zur Handhabung der Vorrichtung angeordnet. Der Rollenkasten 9 weist an seiner Unterseite, also auf der Seite der Griffbolzen 14 und 15 relativ zum Grundkörper 1, einen Referenzpunkt 4 auf, an dem ein erstes Ende eines Maßbandes 3 befestigt ist. An seiner der Unterseite gegenüberliegenden Oberseite weist der Rollenkasten 9 eine Führungsrolle 10 auf, deren Drehachse senkrecht zur Längsachse des Grundkörpers 1 als auch senkrecht zu den Griffbolzen 14 und 15 verläuft. Über diese Führungsrolle 10 wird das Maßband 3 von dem Referenzpunkt 4 kommend zwischen der Führungsrolle 10 und dem Rollenkasten 4 geführt, so dass das Maßband 3 im Bereich des ersten Endes des Grundkörpers 1 eine Schlaufe bildet. Von der Führungsrolle 10 kommend wird das Maßband 3 auf der den Griffbolzen 14 und 15 abgewandten Oberseite des Grundkörpers 1 unter einer an dem Grundkörper 1 im mittleren Bereich bezüglich der Längsachse des Grundkörpers 1 befestigten Messkante 6 hindurchgeführt. Auf der den Griffbolzen 14 und 15 abgewandten Oberseite des Zugelements 2 ist ein flächiger, länglicher Bügel 16 parallel zur Längsachse des Zugelements 2 laufend angeordnet, der an dem der Öffnung 4 abgewandten Ende des Zugelements 2 fest mit dem Zugelement 2 verbunden ist. Auf dem Bügel 16 ist benachbart zur Messkante 6 eine Klemme 13 zur Arretierung des Maßbandes 3 in Richtung der Längsachse des Grundkörpers 1 fest angeordnet. Weiter ist im Bereich des Griffbolzens 15 auf dem Bügel 16 eine Bandlaufrolle 11 derart angeordnet, dass die Drehachse der Bandlaufrolle 11 parallel zur Drehachse der Führungsrolle 10 verläuft. Von der Messkante 6 wird das Maßband 3 also durch die Klemme 13 geführt. Der sich auf der der Messkante 6 abgewandten Seite der Klemme 13 befindliche Teil des Maßbands 3 wird als zweites Ende 3b des Maßbands 3 auf der Bandlaufrolle 11 aufgerollt. Hierzu verfügt die Bandlaufrolle 11 über eine Einzugseinrichtung .

Mittels der vorbeschriebenen Vorrichtung lässt sich ein Verfahren zur Bestimmung des Umfangs beispielsweise eines Unterarms wie folgt durchführen. Zunächst wird die Gasdruckfeder vorgespannt, indem das Zugelement 2 in den Grundkörper 1 hineingedrückt wird. Das Zugelement 2 verrastet mit der Verriegelungseinrichtung 12, sodass das Zugelement 2 nicht aufgrund des Druckes der Gasdruckfeder zurück bewegt wird.

Anschließend wird das Maßband 3 als Schlaufe um den Unterarm gelegt. Hierbei ist die Klemme 13 geöffnet, sodass die Größe der Schlaufe beliebig variierbar ist. Nach Anlegen der Schlaufe verkleinert sich die Schlaufe aufgrund der Einzugseinrichtung der Bandlaufrolle 11. Sobald das Maßband 3 eng am Unterarm anliegt wird die Klemme 13 geschlossen, sodass die Schlaufe nicht mehr vergrößert werden kann.

Als nächstes wird die Verriegelungseinrichtung 12 gelöst. Aufgrund des Druckes der Gasdruckfeder wird das Zugelement 2 entlang der Längsachse des Grundkörpers 1 durch die Öffnung 4 aus dem Grundkörper 1 herausbewegt. Hierdurch wirkt eine Zugkraft auf das Maßband 3, wodurch sich die Größe der Schlaufe weiter verringert. Hierdurch wird das Gewebe des Unterarms mit einer durch die Federkraft der Gasdruckfeder vorbestimmten Kraft leicht zusammengedrückt. Der Umfang des Unterarms kann nun an der Messkante 6 abgelesen werde.

Neben der Bestimmung des Umfangs eines Körperteils eignet sich die erfindungsgemäße Vorrichtung auch allgemein für die Messung eines Umfangs von Gegenständen, bei der ein definierter Zug erforderlich ist.

## Patentansprüche

1. Vorrichtung zur Bestimmung des Umfangs eines Körperteils mit
einem Grundkörper (1),
einem gegenüber dem Grundkörper (1) in einer ersten Richtung verschiebbar gelagerten Zugelement (2),
einem Band (3), dessen erstes Ende (3a) an einem Referenzpunkt (4) des Grundkörpers (1) fest mit dem Grundkörper (1) verbunden ist und dessen zweites Ende (3b) an einer Arretierungsstelle (5) des Zugelements (2) an dem Zugelement (2) arretierbar ist, und
einer Messvorrichtung zur direkten oder indirekten Bestimmung der Länge des Bandes (3) zwischen dem Referenzpunkt (4) und einer Messstelle (6) und hieraus des Umfangs eines von dem Band (3) umfaßten Körperteils,
wobei die Vorrichtung eine Zugerzeugungsvorrichtung zur Ausübung einer von der Verschiebung des Zugelements (2) unabhängigen einheitlichen Zugkraft parallel zu der ersten Richtung über das Zugelement (2) auf das Band (3) aufweist, **dadurch gekennzeichnet, dass** die Zugerzeugungsvorrichtung eine Gasdruck- oder eine Öldruckfeder aufweist.

2. Vorrichtung nach dem vorhergehenden Anspruch, **dadurch gekennzeichnet, dass** die Zugerzeugungsvorrichtung derart ausgebildet ist, daß sie eine Kraft zwischen 5 N und 80 N, vorteilhafterweise zwischen 5 N und 20 N, vorteilhafterweise von 10 N auf die Zugvorrichtung (2) ausübt.

3. Vorrichtung nach einem der vorhergehenden Ansprüche, **dadurch gekennzeichnet, dass** der Grundkörper (1) als länglicher Hohlkörper ausgebildet ist, an dessen erstem Ende das erste Ende (3a) des Bands (3) befestigt ist und dessen dem ersten Ende gegenüberliegendes zweites Ende eine Öffnung (8) aufweist, und in dem die Zugerzeugungsvorrichtung derart gelagert ist, dass die Richtung der Zugkraft und die Längsachse des Grundkörpers (1) parallel zueinander liegen.

4. Vorrichtung nach dem vorhergehenden Anspruch 3, **dadurch gekennzeichnet, dass** das Zugelement (2) länglich ausgebildet ist und zumindest teilweise innerhalb des Grundkörpers (2) in Richtung von dessen Längsachse durch die Öffnung (8) hindurch verschiebbar gelagert ist und durch die Zugerzeugungsvorrichtung mit einer Druckkraft relativ zum Grundkörper (1) beaufschlagbar ist.

5. Vorrichtung nach einem der vorhergehenden Ansprüche, **dadurch gekennzeichnet, dass** die Vorrichtung eine Verriegelungseinrichtung (12) zur Arretierung des Zugelements (2) in einem vorgespannten Zustand aufweist, in dem das Zugelement (2) keine Zugkraft auf das Band (3) ausübt.

6. Vorrichtung nach dem vorhergehenden Anspruch 5, **dadurch gekennzeichnet, dass** die Verriegelungseinrichtung (12) ein Entriegelungselement aufweist, durch welches das Zugelement (2) von dem vorgespannten Zustand in einen Zustand bringbar ist, in dem das Zugelement (2) eine Zugkraft auf das Band (3) ausübt.

7. Vorrichtung nach einem der vorhergehenden Ansprüche, **dadurch gekennzeichnet, dass** an dem Zugelement (2) eine Klemme (13) zum Arretieren des zweiten Endes (3b) des Bands (3) fest angeordnet ist.

8. Vorrichtung nach einem der vorhergehenden Ansprüche, **dadurch gekennzeichnet, dass** der Grundkörper (1) Führungsrollen (10) oder Gleitflächen zur Führung des Bands (3) aufweist.

9. Vorrichtung nach einem der vorhergehenden Ansprüche, **dadurch gekennzeichnet, dass** das Band (3) ein Maßband, eine Schnur oder eine Schnur mit einer Messskala ist.

10. Vorrichtung nach einem der vorhergehenden Ansprüche, **dadurch gekennzeichnet, dass** die Messvorrichtung eine digitale Messeinheit aufweist.

11. Verfahren zur Bestimmung eines Umfangs eines Körperteils mit einer Vorrichtung nach einem der vorhergehenden Ansprüche,
**dadurch gekennzeichnet,**
**dass** das Band (3) um ein Körperteil gelegt wird,
das zweite Ende (3b) des Bands (3) an dem Zugelement (2) derart arretiert wird, dass das Band (3) an dem Körperteil anliegt,
mittels der Zugerzeugungsvorrichtung über das Zugelement (2) eine Zugkraft auf das Band (3) ausgeübt wird,
und mittels der Messvorrichtung die Länge des Bands (3) zwischen dem Referenzpunkt (4) und der Messstelle (6) und daraus der Umfang des Körperteils bestimmt wird.

12. Verfahren nach dem vorhergehenden Anspruch, **dadurch gekennzeichnet, dass** die Zugerzeugungsvorrichtung vor dem Anlegen des Bandes (3) an das Körperteil vorgespannt und mittels der Verriegelungseinrichtung (12) arretiert wird und zur Aufbringung der Zugkraft auf das Band (3) das Entriegelungselement betätigt wird.

## Claims

1. A device for determining the circumference of a body part having a base body (1),
a tension element (2) mounted such that it is displaceable relative to the base body (1) in a first direction,
a tape (3) whereof the first end (3a) is fixedly connected to the base body (1) at a reference point (4) of the base body (1) and whereof the second end (3b) may be arrested on the tension element (2) at an arresting point (5) of the tension element (2), and a measuring device for directly or indirectly determining the length of the tape (3) between the reference point (4) and a measuring point (6) and, from this, the circumference of a body part encompassed by the tape (3),
wherein the device has a tension generating device for exerting a uniform tensile force on the tape (3) parallel to the first direction via the tension element (2), which tensile force is independent of the displacement of the tension element (2), **characterised in that** the tension generating device has a gas pressure or oil pressure spring.

2. The device according to the preceding claim, **characterised in that** the tension generating device is formed in such a way that it exerts a force between 5 N and 80 N, advantageously between 5 N and 20 N, advantageously of 10 N, on the tension device (2).

3. The device according to one of the preceding claims, **characterised in that** the base body (1) is formed as an elongated hollow body, to the first end of which the first end (3a) of the tape (3) is fastened and whereof the second end, opposite the first end, has an opening (8) and in which the tension generating device is mounted in such a way that the direction of the tensile force and the longitudinal axis of the base body (1) are parallel to one another.

4. The device according to the preceding Claim 3, **characterised in that** the tension element (2) is elongated in form and is mounted such that it is displaceable at least partly within the base body (2) in the direction of the longitudinal axis thereof through the opening (8) and may be acted upon by a pressure force relative to the base body (1) by means of the tension generating device.

5. The device according to one of the preceding claims, **characterised in that** the device has a locking mechanism (12) for arresting the tension element (2) in a pre-stressed state in which the tension element (2) does not exert a tensile force on the tape (3).

6. The device according to the preceding Claim 5, **characterised in that** the locking mechanism (12) has an unlocking element by means of which the tension element (2) may be brought from the pre-stressed state to a state in which the tension element (2) exerts a tensile force on the tape (3).

7. The device according to one of the preceding claims, **characterised in that** a clip (13) for arresting the second end (3b) of the tape (3) is fixedly arranged on the tension element (2).

8. The device according to one of the preceding claims, **characterised in that** the base body (1) has guide rollers (10) or slide faces for guiding the tape (3).

9. The device according to one of the preceding claims, **characterised in that** the tape (3) is a measuring tape, a band or a band with a measuring scale.

10. The device according to one of the preceding claims, **characterised in that** the measuring device has a digital measuring unit.

11. A method for determining a circumference of a body part by means of a device according to one of the preceding claims,
**characterised in that**
the tape (3) is placed around a body part,
the second end (3b) of the tape (3) is arrested on the tension element (2) in such a way that the tape (3) lies against the body part,
a tensile force is exerted on the tape (3) via the tension element (2) by means of the tension generating device,
and, by means of the measuring device, the length of the tape (3) between the reference point (4) and the measuring point (6) is determined and, from this, the circumference of the body part.

12. The method according to the preceding claim, **characterised in that**, before the tape (3) is placed against the body part, the tension generating device is pre-stressed and arrested by means of the locking mechanism (12) and, to apply the tensile force to the tape (3), the unlocking element is actuated.

## Revendications

1. Dispositif destiné à déterminer le pourtour d'une partie corporelle avec
un corps de base (1),
un élément de traction (2) supporté de façon coulissante dans une première direction par rapport au corps de base (1),
une bande (3) dont la première extrémité (3a) est raccordée fixement au corps de base (1) au niveau d'un point de référence (4) du corps de base (1) et dont la deuxième extrémité (3b) peut être bloquée sur l'élément de traction (2) au niveau d'un emplacement de blocage (5) de l'élément de traction (2), et
un dispositif de mesure destiné à la détermination directe ou indirecte de la longueur de la bande (3) entre le point de référence (4) et un emplacement de mesure (6) et, à partir de là, du pourtour d'une partie corporelle entourée par la bande (3),
le dispositif présentant un dispositif de production de traction destiné à exercer sur la bande (3), par le biais de l'élément de traction (2), une force de traction uniforme indépendante du coulissement de l'élément de traction (2) parallèlement à la première direction,
**caractérisé en ce que**
le dispositif de production de traction présente un ressort de pression à gaz ou à huile.

2. Dispositif selon la revendication précédente, **caractérisé en ce que** le dispositif de production de traction est constitué de telle sorte qu'il exerce sur le dispositif de traction (2) une force comprise entre 5 N et 80 N, de préférence entre 5 N et 20 N, de préférence égale à 10 N.

3. Dispositif selon l'une des revendications précédentes, **caractérisé en ce que** le corps de base (1) est constitué en tant que corps creux allongé à la première extrémité duquel est fixée la première extrémité (3a) de la bande (3),et dont la deuxième extrémité opposée à la première extrémité présente une ouverture (8), et dans lequel le dispositif de production de traction est supporté de telle sorte que la direction de la force de traction et l'axe longitudinal du corps de base (1) sont parallèles entre eux.

4. Dispositif selon la revendication précédente 3, **caractérisé en ce que** l'élément de traction (2) est constitué de façon allongée et est supporté au moins partiellement à l'intérieur du corps de base (2) de façon coulissante dans la direction de son axe longitudinal à travers l'ouverture (8) et peut être exposé par le dispositif de production de traction à une force de pression relativement au corps de base (1).

5. Dispositif selon l'une des revendications précédentes, **caractérisé en ce que** le dispositif présente un équipement de verrouillage (12) destiné au blocage de l'élément de traction (2) dans un état précontraint dans lequel l'élément de traction (2)n'exerce pas de force de traction sur la bande (3).

6. Dispositif selon la revendication précédente 5, **caractérisé en ce que** l'équipement de verrouillage (12) présente un élément de déverrouillage par lequel l'élément de traction (2) peut être conduit de l'état précontraint vers un état dans lequel l'élément de traction (2) exerce une force de traction sur la bande (3).

7. Dispositif selon l'une des revendications précédentes, **caractérisé en ce que**, sur l'élément de traction (2), une pince (13) est disposée fixement pour le blocage de la deuxième extrémité (3b) de la bande (3).

8. Dispositif selon l'une des revendications précédentes, **caractérisé en ce que** le corps de base (1) présente des galets de guidage (10) ou des surfaces de glissement pour le guidage de la bande (3).

9. Dispositif selon l'une des revendications précédentes, **caractérisé en ce que** la bande (3) est un mètre ruban, une ficelle ou une ficelle avec une échelle de mesure.

10. Dispositif selon l'une des revendications précédentes, **caractérisé en ce que** le dispositif de mesure présente une unité de mesure numérique.

11. Procédé destiné à déterminer un pourtour d'une partie corporelle avec un dispositif selon l'une des revendications précédentes,
**caractérisé en ce que**
la bande (3) est placée autour d'une partie corporelle,
la deuxième extrémité (3b) de la bande (3) est bloquée sur l'élément de traction (2) de telle sorte que la bande (3) est adjacente à la partie corporelle,
une force de traction est exercée sur la bande (3) au moyen du dispositif de production de traction par le biais de l'élément de traction (2), et, au moyen du dispositif de mesure, la longueur de la bande (3) entre le point de référence (4) et l'emplacement de mesure (6) est déterminée, et le pourtour de la partie corporelle est déterminé à partir de cela

12. Procédé selon la revendication précédente, **caractérisé en ce que** le dispositif de production de traction est précontraint avant la pose de la bande (3) sur la partie corporelle et est bloqué au moyen de l'équipement de verrouillage (12), et l'élément de déverrouillage est actionné pour l'application de la force de traction sur la bande (3).
